(19) Europäisches Patentamt
European Patent Office
Office européen des brevets

(11) Veröffentlichungsnummer: **0 033 825**
**B1**

# (12) EUROPÄISCHE PATENTSCHRIFT

(45) Veröffentlichungstag der Patentschrift: **14.03.84**

(51) Int. Cl.³: **A 61 K 31/66**, C 07 F 9/113, C 07 F 9/173, C 07 F 9/24, C 07 F 9/40

(21) Anmeldenummer: **81100027.2**

(22) Anmeldetag: **07.01.81**

(54) **2-Cyano-vinyl-(thiono)(thiol)-phospor- bzw. -phosphonsäure-Derivate als endoparasitizide Mittel.**

(30) Priorität: **17.01.80 DE 3001519**

(43) Veröffentlichungstag der Anmeldung: **19.08.81 Patentblatt 81/33**

(45) Bekanntmachung des Hinweises auf die Patenterteilung: **14.03.84 Patentblatt 84/11**

(84) Benannte Vertragsstaaten: **DE FR GB**

(56) Entgegenhaltungen:
**DE - A - 2 030 509**
**DE - A - 2 049 695**
**DE - A - 2 302 273**
**DE - A - 2 409 462**
**DE - A - 2 535 514**
**US - A - 3 536 791**
**US - A - 3 553 322**

(73) Patentinhaber: **BAYER AG, Konzernverwaltung RP Patentabteilung, D-5090 Leverkusen 1 Bayerwerk (DE)**

(72) Erfinder: **Thomas, Herbert, Dr., Bergerheide 82b, D-5600 Wuppertal 1 (DE)**
Erfinder: **Andrews, Peter, Dr., Gellertweg 2, D-5600 Wuppertal 1 (DE)**
Erfinder: **Hofer, Wolfgang, Dr., Pahlkestrasse 59, D-5600 Wuppertal 1 (DE)**
Erfinder: **Maurer, Fritz, Dr., Roeberstrasse 8, D-5600 Wuppertal 1 (DE)**
Erfinder: **Riebel, Hans-Jochem, Dr., In der Beek 92, D-5600 Wuppertal 1 (DE)**
Erfinder: **Rohe, Lothar, Dr., Damaschkeweg 75, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## 2-Cyano-vinyl-(thiono) (thiol)-phosphor- bzw. -phosphonsäure-Derivate als endoparasitizide Mittel

Die Erfindung betrifft die Verwendung von weitgehend bekannten 2-Cyano-vinyl-(thiono) (thiol)-phosphor- bzw. -phosphonsäureestern bzw. -esteramiden als Endoparasitizide, insbesondere als Anthelmintika.

Es ist bereits bekannt, daß bestimmte 2-Cyano-vinyl-thionophosphorsäureester, wie z. B. O-[2-Cyano-1-(3-methyl-phenyl)-vinyl]- und O-[2-Cyano-1-(2-brom-phenyl)-vinyl]-O,O-diethyl-thionophosphorsäureester zur Bekämpfung von pflanzenschädigenden Insekten und Milben verwendet werden können (vergleiche DE-OS 2 030 509).

Weiter ist bekannt, daß sich durch die jahrelange Anwendung bestimmter Benzimidazolderivate, wie z. B. 2-(4-Thiazolyl)-benzimidazol (Thiabendazole), Methyl-(5-phenylthio-benzimidazol-2-yl)-carbamat (Fenbendazole), 5-N-Butyl-2-methoxycarbonylaminobenzimidazol (Parbendazole) und Methyl-(5-propylthio-benzimidazol-2-yl)-carbamat (Albendazole), die für die Bekämpfung der Nematoden der Nutztiere als Mittel der Wahl gelten, gegen Benzimidazol-Anthelmintika resistente Parasitenstämme herausgebildet haben [vergleiche Adv. Pharmacol. Chemoth. 16 (1979), 89—127].

Es wurde nun gefunden, daß die weitgehend bekannten 2-Cyano-vinyl-(thiono) (thiol)-phosphor- bzw. -phosphonsäureester bzw. -esteramide der allgemeinen Formel I

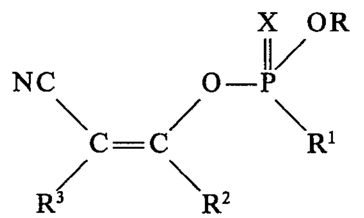

(I)

in welcher

X für Sauerstoff oder Schwefel,
R für Alkyl($C_1-C_4$),
$R^1$ für Alkyl($C_1-C_4$), Phenyl, Alkoxy($C_1-C_4$), Alkylthio($C_1-C_4$) oder Alkylamino($C_1-C_4$),
$R^2$ für gegebenenfalls durch Halogen, Cyano, Nitro oder durch gegebenenfalls halogen-substituierte Substituenten der Reihe Alkyl($C_1-C_4$), Alkoxy($C_1-C_4$) oder Alkylthio($C_1-C_4$) substituiertes Phenyl oder Naphthyl und
$R^3$ für Wasserstoff oder Alkyl($C_1-C_4$) steht,

sehr gut zur Bekämpfung von Endoparasiten verwendet werden können.

Überraschenderweise sind die erfindungsgemäß zu verwendenden 2-Cyano-vinyl-(thiono) (thiol)-phosphorsäure- bzw. -phosphonsäureester bzw. -esteramide, insbesondere gegen solche Endoparasiten, die gegen die als Mittel der Wahl geltenden bekannten Benzimidazol-Anthelmintika resistent geworden sind, voll wirksam. Es besteht also keine Kreuzresistenz zwischen Benzimidazolderivaten und den erfindungsgemäß zu verwendenden Verbindungen.

Die erfindungsgemäße Verwendung der Wirkstoffe der allgemeinen Formel I stellt somit eine wertvolle Bereicherung der Tiermedizin dar.

Die allgemeine Formel I stellt von den möglichen geometrischen Isomeren die Z-Isomeren dar. Die Erfindung betrifft jedoch auch die entsprechenden E-Isomeren und Gemische aus Z- und E-Isomeren.

Die erfindungsgemäß zu verwendenden 2-Cyano-vinyl-(thiono) (thiol)-phosphorsäure- bzw. -phosphonsäureester bzw. -esteramide sind durch die allgemeine Formel I definiert. Vorzugsweise stehen darin

X für Sauerstoff oder Schwefel,
R für Methyl, Ethyl oder Propyl,
$R^1$ für Methyl, Ethyl, Phenyl, Methoxy, Ethoxy, n- oder iso-Propoxy, Methylthio, Ethylthio, Propylthio, Methylamino, Ethylamino, n- oder iso-Propylamino,
$R^2$ für gegebenenfalls durch Fluor, Chlor, Brom, Cyano, Nitro, Methyl, Ethyl, n- oder iso-Propyl, Methoxy, und/oder Trifluormethyl substituiertes Phenyl oder Naphthyl und
$R^3$ für Wasserstoff oder Methyl.

Beispiele für die erfindungsgemäß zu verwendenden Verbindungen der allgemeinen Formel I sind nachstehend tabellarisch aufgeführt.

Tabelle: (Z/E-Isomerie nicht festgelegt)

| Nr. | X | R | $R^1$ | $R^2$ | $R^3$ | Physikal. Konst. |
|---|---|---|---|---|---|---|
| 1 | S | $C_2H_5$ | $OC_2H_5$ | $C_6H_5$ | H | $n_D^{28}$ : 1,5411 |
| 2 | S | $C_2H_5$ | $OC_2H_5$ | 2-$CH_3$–$C_6H_4$ | H | $n_D^{25}$ : 1,5388 |
| 3 | S | $C_2H_5$ | $OC_2H_5$ | 4-Cl–$C_6H_4$ | H | $n_D^{21}$ : 1,5665 |
| 4 | S | $CH_3$ | $OCH_3$ | $C_6H_5$ | H | $n_D^{28}$ : 1,5655 |
| 5 | S | n-$C_3H_7$ | $OC_3H_7$-n | $C_6H_5$ | H | $n_D^{25}$ : 1,5262 |
| 6 | S | iso-$C_3H_7$ | $OC_3H_7$-iso | $C_6H_5$ | H | $n_D^{21}$ : 1,5301 |
| 7 | S | $CH_3$ | $C_2H_5$ | $C_6H_5$ | H | $n_D^{25}$ : 1,5684 |
| 8 | S | $C_2H_5$ | $C_2H_5$ | $C_6H_5$ | H | $n_D^{28}$ : 1,5600 |
| 9 | S | $C_2H_5$ | $C_6H_5$ | $C_6H_5$ | H | $n_D^{22}$ : 1,6008 |
| 10 | S | $C_2H_5$ | NH–$CH_3$ | $C_6H_5$ | H | $n_D^{25}$ : 1,5509 |
| 11 | S | $C_2H_5$ | $C_2H_5$ | 2-$CH_3$–$C_6H_4$ | H | $n_D^{25}$ : 1,5455 |
| 12 | S | $C_2H_5$ | $OC_2H_5$ | 3-$CH_3$–$C_6H_4$ | H | $n_D^{23}$ : 1,5274 |
| 13 | S | $C_2H_5$ | $C_2H_5$ | 3-$CH_3$–$C_6H_4$ | H | $n_D^{25}$ : 1,5389 |
| 14 | O | $C_2H_5$ | $OC_2H_5$ | 3-$CH_3$–$C_6H_4$ | H | $n_D^{25}$ : 1,4981 |
| 15 | S | $C_2H_5$ | $OC_2H_5$ | 4-$CH_3$–$C_6H_4$ | H | $n_D^{23}$ : 1,5464 |
| 16 | S | $C_2H_5$ | $C_2H_5$ | 4-$CH_3$–$C_6H_4$ | H | $n_D^{23}$ : 1,5545 |
| 17 | O | $C_2H_5$ | $OC_2H_5$ | 4-$CH_3$–$C_6H_4$ | H | $n_D^{23}$ : 1,5270 |
| 18 | S | $C_2H_5$ | $C_2H_5$ | 4-Cl–$C_6H_4$ | H | $n_D^{21}$ : 1,5755 |
| 19 | O | $C_2H_5$ | $OC_2H_5$ | 4-Cl–$C_6H_4$ | H | $n_D^{22}$ : 1,5387 |
| 20 | S | $C_2H_5$ | $OC_2H_5$ | 3-Cl–$C_6H_4$ | H | $n_D^{25}$ : 1,5529 |
| 21 | S | $C_2H_5$ | $C_2H_5$ | 3-Cl–$C_6H_4$ | H | $n_D^{20}$ : 1,5577 |
| 22 | O | $C_2H_5$ | $OC_2H_5$ | 3-Cl–$C_6H_4$ | H | $n_D^{24}$ : 1,5215 |
| 23 | S | $C_2H_5$ | $OC_2H_5$ | 2-Cl–$C_6H_4$ | H | $n_D^{26}$ : 1,5430 |
| 24 | S | $C_2H_5$ | $C_2H_5$ | 2-Cl–$C_6H_4$ | H | $n_D^{26}$ : 1,5529 |
| 25 | O | $C_2H_5$ | $OC_2H_5$ | 2-Cl–$C_6H_4$ | H | $n_D^{26}$ : 1,5029 |
| 26 | S | $C_2H_5$ | $OC_2H_5$ | 2,5-$Cl_2$–$C_6H_3$ | H | $n_D^{25}$ : 1,5442 |
| 27 | S | $C_2H_5$ | $C_2H_5$ | 2,5-$Cl_2$–$C_6H_3$ | H | $n_D^{23}$ : 1,5647 |
| 28 | S | $C_2H_5$ | $OC_2H_5$ | 2,4-$Cl_2$–$C_6H_3$ | H | $n_D^{24}$ : 1,5506 |
| 29 | S | $C_2H_5$ | $C_2H_5$ | 2,4-$Cl_2$–$C_6H_3$ | H | $n_D^{21}$ : 1,5517 |
| 30 | S | $C_2H_5$ | $OC_2H_5$ | 4-$CH_3O$–$C_6H_4$ | H | $n_D^{22}$ : 1,5562 |
| 31 | S | $C_3H_{7-n}$ | $OC_3H_{7-n}$ | 4-$CH_3O$–$C_6H_4$ | H | $n_D^{22}$ : 1,5498 |

Fortsetzung

| Nr. | X | R | $R^1$ | $R^2$ | $R^3$ | Physikal. Konst. |
|---|---|---|---|---|---|---|
| 32 | S | $C_2H_5$ | $CH_3$ | 4-$CH_3O-C_6H_4$ | H | Smp.: 72°C |
| 33 | S | $C_2H_5$ | $C_2H_5$ | 4-$CH_3O-C_6H_4$ | H | $n_D^{22}$: 1,5810 |
| 34 | S | $C_2H_5$ | $C_6H_5$ | 4-$CH_3O-C_6H_4$ | H | $n_D^{22}$: 1,6142 |
| 35 | O | $C_2H_5$ | $OC_2H_5$ | 4-$CH_3O-C_6H_4$ | H | $n_D^{23}$: 1,5369 |
| 36 | S | $C_2H_5$ | $OC_2H_5$ | 3-$CH_3O-C_6H_4$ | H | $n_D^{20}$: 1,5525 |
| 37 | O | $C_2H_5$ | $C_2H_5$ | 3-$CH_3O-C_6H_4$ | H | $n_D^{25}$: 1,5300 |
| 38 | S | $C_2H_5$ | $C_2H_5$ | 3-$CH_3O-C_6H_4$ | H | $n_D^{25}$: 1,5624 |
| 39 | O | $C_2H_5$ | $OC_2H_5$ | 2-$CH_3O-C_6H_4$ | H | $n_D^{23}$: 1,5410 |
| 40 | S | $C_2H_5$ | $C_2H_5$ | 2-$CH_3O-C_6H_4$ | H | $n_D^{22}$: 1,5397 |
| 41 | S | $CH_3$ | $OCH_3$ | 4-$CH_3-C_6H_4$ | H | Smp.: 85°C |
| 42 | S | $C_2H_5$ | $C_6H_5$ | 4-$Cl-C_6H_4$ | H | $n_D^{24}$: 1,6078 |
| 43 | S | $C_2H_5$ | $C_6H_5$ | 4-$CH_3-C_6H_4$ | H | $n_D^{24}$: 1,5990 |
| 44 | S | $C_3H_7$-iso | $OC_3H_7$-iso | 4-$CH_3-C_6H_5$ | H | $n_D^{24}$: 1,5282 |
| 45 | S | $C_2H_5$ | $C_2H_5$ | 2-$Br-C_6H_4$ | H | $n_D^{24}$: 1,5571 |
| 46 | S | $C_2H_5$ | $OC_2H_5$ | 2-$Br-C_6H_4$ | H | $n_D^{24}$: 1,5525 |
| 47 | S | $C_2H_5$ | $OC_2H_5$ | 2-Cl-4-$C_2H_5S-C_6H_3$ | H | $n_D^{21}$: 1,5952 |
| 48 | S | $C_2H_5$ | $OC_2H_5$ | 2,5-$(CH_3)_2-C_6H_3$ | H | $n_D^{24}$: 1,5303 |
| 49 | S | $C_2H_5$ | $OC_2H_5$ | $C_6H_5$ | $CH_3$ | $n_D^{21}$: 1,5340 |
| 50 | S | $C_2H_5$ | $C_2H_5$ | $C_6H_5$ | $CH_3$ | $n_D^{20}$: 1,5508 |
| 51 | S | $C_2H_5$ | $C_6H_5$ | $C_6H_5$ | $CH_3$ | $n_D^{20}$: 1,5878 |
| 52 | S | $C_2H_5$ | $OC_2H_5$ | 4-$CH_3-C_6H_4$ | $CH_3$ | $n_D^{23}$: 1,5358 |
| 53 | S | $C_2H_5$ | $C_2H_5$ | 4-$CH_3-C_6H_4$ | $CH_3$ | $n_D^{23}$: 1,5492 |
| 54 | S | $C_2H_5$ | $OC_2H_5$ | 4-$CH_3O-C_6H_4$ | $CH_3$ | $n_D^{25}$: 1,5454 |
| 55 | S | $C_2H_5$ | $C_2H_5$ | 4-$CH_3O-C_6H_4$ | $CH_3$ | $n_D^{25}$: 1,5571 |
| 56 | S | $C_2H_5$ | $OC_2H_5$ | 4-$Cl-C_6H_4$ | $CH_3$ | $n_D^{24}$: 1,5450 |
| 57 | S | $C_2H_5$ | $C_2H_5$ | 4-$Cl-C_6H_4$ | $CH_3$ | $n_D^{24}$: 1,5623 |
| 58 | S | $CH_3$ | $OCH_3$ | 4-$Cl-C_6H_4$ | $CH_3$ | $n_D^{24}$: 1,5580 |
| 59 | S | $C_3H_7$-iso | $OC_3H_7$-iso | 4-$Cl-C_6H_4$ | $CH_3$ | $n_D^{24}$: 1,5293 |
| 60 | S | $C_2H_5$ | $OC_2H_5$ | 2-$Br-C_6H_4$ | $CH_3$ | $n_D^{25}$: 1,5453 |
| 61 | S | $C_2H_5$ | $CH_3$ | 2-$Br-C_6H_4$ | $CH_3$ | $n_D^{25}$: 1,5639 |
| 62 | S | $C_2H_5$ | $SC_3H_7$-n | $C_6H_5$ | H | $n_D^{22}$: 1,5683 |

Fortsetzung

| Nr. | X | R | $R^1$ | $R^2$ | $R^3$ | Physikal. Konst. |
|---|---|---|---|---|---|---|
| 63 | S | $C_2H_5$ | $SC_3H_7$-n | 4-Cl–$C_6H_4$ | H | $n_D^{22}$ : 1,5788 |
| 64 | S | $C_2H_5$ | $SC_3H_7$-n | 3-$CH_3$–$C_6H_4$ | H | $n_D^{25}$ : 1,5600 |
| 65 | S | $C_2H_5$ | $SC_3H_7$-n | 2-$CH_3$–$C_6H_4$ | H | $n_D^{25}$ : 1,5991 |
| 66 | S | $C_2H_5$ | $SC_3H_7$-n | 4-$CH_3$–$C_6H_4$ | H | $n_D^{25}$ : 1,5669 |
| 67 | S | $C_2H_5$ | $SC_3H_7$-n | 3-$CH_3O$–$C_6H_4$ | H | $n_D^{25}$ : 1,5708 |
| 68 | S | $C_2H_5$ | $SC_3H_7$-n | 2-$CH_3O$–$C_6H_4$ | H | $n_D^{25}$ : 1,5623 |
| 69 | S | $C_2H_5$ | $SC_3H_7$-n | 4-$CH_3O$–$C_6H_4$ | H | $n_D^{23}$ : 1,5714 |
| 70 | S | $C_2H_5$ | $SC_3H_7$-n | 3-Cl–$C_6H_4$ | H | $n_D^{25}$ : 1,5691 |
| 71 | S | $C_2H_5$ | $SC_3H_7$-n | 2-Cl–$C_6H_4$ | H | $n_D^{23}$ : 1,5512 |
| 72 | S | $C_2H_5$ | $SC_3H_7$-n | 2-Br–$C_6H_4$ | H | $n_D^{23}$ : 1,5808 |
| 73 | S | $C_2H_5$ | $SC_3H_7$-n | 3-$CF_3$–$C_6H_4$ | H | $n_D^{24}$ : 1,5361 |
| 74 | S | $C_2H_5$ | $SC_3H_7$-n | 1-Naphthyl | H | $n_D^{24}$ : 1,5601 |
| 75 | S | $C_2H_5$ | $SC_3H_7$-n | 4-Br–$C_6H_4$ | H | $n_D^{19}$ : 1,5798 |
| 76 | S | $C_2H_5$ | $SC_3H_7$-n | 4-F–$C_6H_4$ | H | $n_D^{19}$ : 1,5590 |
| 77 | S | $C_2H_5$ | $SC_3H_7$-n | 4-CN–$C_6H_4$ | H | $n_D^{25}$ : 1,5706 |
| 78 | S | $C_2H_5$ | $SC_3H_7$-n | 2,4-$Cl_2$–$C_6H_3$ | H | $n_D^{21}$ : 1,5680 |
| 79 | S | $C_2H_5$ | $SC_3H_7$-n | 2,5-$Cl_2$–$C_6H_3$ | H | $n_D^{21}$ : 1,5778 |
| 80 | S | $C_2H_5$ | $SC_3H_7$-n | 2,4,6-$Cl_3$–$C_6H_2$ | H | $n_D^{25}$ : 1,5810 |
| 81 | S | $C_2H_5$ | $SC_3H_7$-n | 2,4-$(CH_3)_2$–$C_6H_3$ | H | $n_D^{25}$ : 1,5609 |
| 82 | S | $C_2H_5$ | $SC_3H_7$-n | 2,4-($C_3H_7$-iso)$_2$–$C_6H_3$ | H | $n_D^{21}$ : 1,5450 |
| 83 | S | $C_2H_5$ | $SC_3H_7$-n | 3,4-$Cl_2$–$C_6H_3$ | H | $n_D^{18}$ : 1,5853 |
| 84 | S | $C_2H_5$ | $SC_3H_7$-n | 3,4-$Br_2$–$C_6H_3$ | H | $n_D^{23}$ : 1,5972 |
| 85 | S | $C_2H_5$ | $SC_3H_7$-n | 2,3,4-$Cl_3$–$C_6H_2$ | H | $n_D^{26}$ : 1,5838 |
| 86 | S | $C_2H_5$ | $SC_3H_7$-n | 2,5-$(CH_3)_2$–$C_6H_3$ | H | $n_D^{22}$ : 1,5548 |
| 87 | S | $C_2H_5$ | $SC_3H_7$-n | $C_6H_5$ | $CH_3$ | $n_D^{24}$ : 1,5608 |
| 88 | S | $C_2H_5$ | $SC_3H_7$-n | 2-Naphthyl | H | $n_D^{24}$ : 1,6147 |
| 89 | S | $C_2H_5$ | $OC_3H_7$-n | 2-$CH_3$–$C_6H_4$ | H | $n_D^{25}$ : 1,5320 |
| 90 | S | $C_2H_5$ | $OC_3H_7$-n | 2-Cl–$C_6H_4$ | H | $n_D^{23}$ : 1,5427 |
| 91 | S | $C_2H_5$ | $OC_3H_7$-n | 2-$CH_3O$–$C_6H_4$ | H | $n_D^{25}$ : 1,5350 |
| 92 | S | $C_2H_5$ | $OC_3H_7$-n | 2,5-$Cl_2$–$C_6H_3$ | H | $n_D^{24}$ : 1,5245 |
| 93 | S | $C_2H_5$ | $OC_3H_7$-n | 4-F–$C_6H_4$ | H | $n_D^{23}$ : 1,5190 |

Fortsetzung

| Nr. | X | R | $R^1$ | $R^2$ | $R^3$ | Physikal. Konst. |
|---|---|---|---|---|---|---|
| 94 | S | $C_2H_5$ | $OC_3H_7$-n | 2,4-$Cl_2$—$C_6H_3$ | H | $n_D^{23}$ : 1,5407 |
| 95 | S | $C_2H_5$ | $OC_3H_7$-n | 2,5-$(CH_3)_2$—$C_6H_3$ | H | $n_D^{22}$ : 1,5272 |
| 96 | S | $C_2H_5$ | $OC_3H_7$-n | 2-F-5-Cl—$C_6H_3$ | H | $n_D^{24}$ : 1,5372 |

Die Wirkstoffe der allgemeinen Formel I sind weitgehend bekannt (vergleiche die deutschen Offenlegungsschriften 2 030 509, 2 049 695, 2 302 273 und 2 409 462).

Einzelne der erfindungsgemäß zu verwendenden Verbindungen der allgemeinen Formel I sind noch nicht in der Literatur beschrieben. Sie können jedoch nach bekannten Verfahren in einfacher Weise hergestellt werden. Man erhält sie beispielsweise, wenn man (Thio)phosphorsäureesterhalogenide, wie z. B. O,O-Diethyl-thionophosphorsäurediesterchlorid, mit $\omega$-Cyanacetophenonen (Benzoylacetonitrilen), wie z. B. $\omega$-Cyan-2,5-dimethyl-acetophenon, gegebenenfalls in Gegenwart eines Säureakzeptors, wie z. B. Kaliumcarbonat, und gegebenenfalls unter Verwendung eines Verdünnungsmittels, wie z. B. Acetonitril, bei Temperaturen zwischen 10 und 80° C umsetzt.

Die Verbindungen der allgemeinen Formel I isoliert man beispielsweise, indem man das Reaktionsgemisch mit Wasser verdünnt, mit einem mit Wasser nicht mischbaren organischen Lösungsmittel, wie z. B. Toluol, extrahiert, die organische Phase mit Wasser wäscht, trocknet, filtriert und vom Filtrat das Lösungsmittel unter vermindertem Druck sorgfältig abdestilliert.

Die erfindungsgemäß zu verwendenden Wirkstoffe können, wie bereits erwähnt, zur Bekämpfung von Endoparasiten verwendet werden.

Die Verbindungen gemäß der allgemeinen Formel I zeigen eine überraschend gute Wirkung gegen Haemonchus contortus, besonders auch gegen solche Stämme, die gegen Benzimidazol-Anthelmintica resistent sind.

Die Wirkung wurde im Tierversuch nach oraler Applikation bei stark mit Parasiten befallenen Versuchstieren geprüft. Die angewendeten Dosierungen wurden sehr gut von den Versuchstieren vertragen.

Die Wirkstoffe gemäß der allgemeinen Formel I können als Anthelmintika sowohl in der Human- als auch in der Veterinärmedizin verwendet werden.

Sie können in bekannter Weise in die üblichen Formulierungen übergeführt werden.

Sie können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elixiere, Sirup, Pasten und dergleichen in Betracht. Derartige Träger umfassen feste Verdünnungsmittel oder Füllstoffe, ein steriles, wäßriges Medium sowie verschiedene nicht-toxische organische Lösungsmittel. Selbstverständlich können die für eine orale Verabreichung in Betracht kommenden Tabletten und dergleichen mit Süßstoffzusatz und ähnlichem versehen werden. Die therapeutisch wirksame Verbindung soll im vorgenannten Fall in einer Konzentration von etwa 0,5 bis 90 Gew.-% der Gesamtmischung vorhanden sein, d. h. in Mengen, die ausreichend sind, um den obengenannten Dosierungsspielraum zu erreichen.

Die Formulierungen werden in bekannter Weise hergestellt, z. B. durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt: Wasser, nichttoxische organische Lösungsmittel, wie Paraffine (z. B. Erdölfraktionen), pflanzliche Öle (z. B. Erdnuß-/Sesamöl), Alkohole (z. B. Äthylalkohol, Glycerin), Glykole (z. B. Propylenglykol, Polyäthylenglykol) und Wasser; feste Trägerstoffe, wie natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker); Emulgiermittel, wie nichtionogene und anionische Emulgatoren (z. B. Polyoxyäthylen-Fettsäure-Ester, Polyoxyäthylen-Fettalkohol-Äther, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Im Falle der oralen Anwendung können Tabletten selbstverständlich außer den genannten Trägerstoffen auch Zusätze, wie Natriumcitrat, Calciumcarbonat und Dicalciumphosphat, zusammen mit verschiedenen Zuschlagstoffen, wie Stärke, vorzugsweise Kartoffelstärke, Gelatine, enthalten. Weiterhin können Gleitmittel, wie Magnesiumstearat, Natriumlaurylsulfat und Talkum, zum Tablettieren mitverwendet werden.

Im Falle wäßriger Suspensionen und/oder Elixieren, die für orale Anwendungen gedacht sind, können die Wirkstoffe außer mit den obengenannten Hilfsstoffen mit verschiedenen Geschmacksauf-

besserern oder Farbstoffen versetzt werden.

Für den Fall der parenteralen Anwendung können Lösungen der Wirkstoffe unter Verwendung geeigneter flüssiger Trägermaterialien eingesetzt werden.

Die Wirkstoffe können in Kapseln, Tabletten, Pastillen, Dragees, Ampullen usw. auch in Form von Dosierungseinheiten enthalten sein, wobei jede Dosierungseinheit so angepaßt ist, daß sie eine einzelne Dosis des aktiven Bestandteils liefert.

Die Wirkstoffe gemäß der allgemeinen Formel I können in üblicher Weise angewendet werden. Die Applikation erfolgt vorzugsweise oral, eine parenterale, insbesondere subkutane Applikation sind jedoch ebenfalls möglich.

Im allgemeinen hat es sich als vorteilhaft erwiesen, Mengen von etwa 0,1 bis etwa 50 mg der Verbindungen je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen. Für die Applikation in der Human- und Veterinärmedizin ist der gleiche Dosierungsspielraum vorgesehen. Sinngemäß gelten auch die weiteren vorstehenden Ausführungen.

Die anthelmintische Wirkung der Wirkstoffe gemäß der allgemeinen Formel I sei anhand des folgenden Anwendungsbeispiels näher erläutert.

Beispiel A

Haemonchus contortus/Schaf

Experimentell mit normal empfindlichen bzw. mit gegen Benzimidazol-Anthelmintika resistenten Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva minima) sind aus der nachfolgenden Tabelle ersichtlich:

| Geprüfte Wirkstoffe | Dosis effectiva minima (Red. ≥ 90%) in mg/kg | |
|---|---|---|
| | Normal empfindlicher Stamm | Resistenter Stamm |
| Verbindung Nr. 12 | 1 | 1 |
| Verbindung Nr. 89 | 2,5 | 2,5 |
| Verbindung Nr. 46 | 1 | 1 |
| Vergleichsverbindungen: | | |
| Thiabendazol | 25 | 250 |
| Parbendazol | 1 | 10 |
| Albendazol | 0,5 | 5 |
| Fenbendazol | 0,5 | 2,5 |

Herstellungsbeispiel

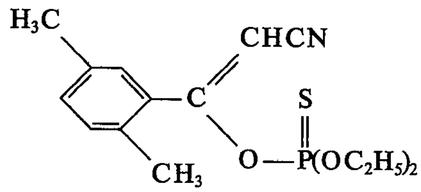

18,8 g O,O-Diethyl-thionophosphorsäurediesterchlorid werden bei 40°C zu einer Mischung aus 17,3 g ω-Cyan-2,5-dimethyl-acetophenon, 13,8 g Kaliumcarbonat und 300 ml Acetonitril tropfenweise gegeben. Das komplette Reaktionsgemisch wird 3 Stunden bei 40—50°C gerührt, dann auf Wasser gegossen und mit Toluol extrahiert. Die Toluolphase wird mit wäßriger Hydrogencarbonatlösung gewaschen, getrocknet und filtriert. Vom Filtrat wird das Lösungsmittel bei mäßig erhöhter Temperatur unter vermindertem Druck sorgfältig abdestilliert. Man erhält 26 g (80% der Theorie) O,O-Diethyl-O-[2-cyano-1-(2,5-dimethyl-phenyl)-vinyl]-thionophosphorsäureester als braunstichigen öligen Rückstand vom Brechungsindex $n_D^{24}$: 1,5303.

Die übrigen Verbindungen in der Tabelle (oben) sind literaturbekannt.

## Patentansprüche

1. Endoparasitizides Mittel, gekennzeichnet durch einen Gehalt an mindestens einer Verbindung der allgemeinen Formel I

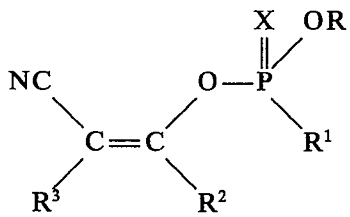

(I)

in welcher

- X für Sauerstoff oder Schwefel,
- R für Alkyl($C_1$—$C_4$),
- $R^1$ für Alkyl($C_1$—$C_4$), Phenyl, Alkoxy($C_1$—$C_4$), Alkylthio($C_1$—$C_4$) oder Alkylamino($C_1$—$C_4$),
- $R^2$ für gegebenenfalls durch Halogen, Cyano, Nitro oder durch gegebenenfalls halogen-substituierte Substituenten der Reihe Alkyl($C_1$—$C_4$), Alkoxy($C_1$—$C_4$) oder Alkylthio($C_1$—$C_4$) substituiertes Phenyl oder Naphthyl und
- $R^3$ für Wasserstoff oder Alkyl($C_1$—$C_4$) steht.

2. Verfahren zur Herstellung eines endoparasitiziden Mittels, dadurch gekennzeichnet, daß man mindestens eine Verbindung der allgemeinen Formel I in Anspruch 1 mit inerten, nichttoxischen, pharmazeutisch geeigneten Trägerstoffen vermischt.

3. Verbindungen der allgemeinen Formel I

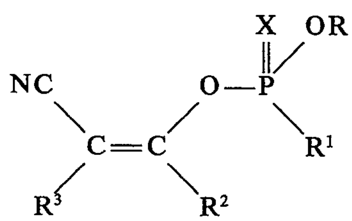

(I)

in welcher

- X für Sauerstoff oder Schwefel,
- R für Alkyl($C_1$—$C_4$),
- $R^1$ für Alkyl($C_1$—$C_4$), Phenyl, Alkoxy($C_1$—$C_4$), Alkylthio($C_1$—$C_4$) oder Alkylamino($C_1$—$C_4$),
- $R^2$ für gegebenenfalls durch Halogen, Cyano, Nitro oder durch gegebenenfalls halogen-substituierte Substituenten der Reihe Alkyl($C_1$—$C_4$), Alkoxy($C_1$—$C_4$) oder Alkylthio($C_1$—$C_4$) substituiertes Phenyl oder Naphthyl und
- $R^3$ für Wasserstoff oder Alkyl($C_1$—$C_4$) steht,

zur Verwendung bei der Bekämpfung von Endoparasiten.

4. Verbindung der Formel

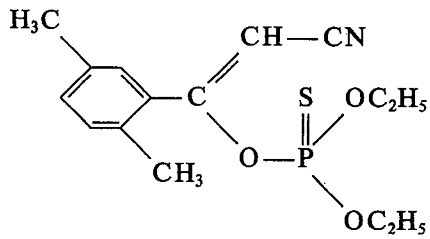

5. Verfahren zur Herstellung der Verbindung der Formel gemäß Anspruch 4, dadurch gekennzeichnet, daß man in an sich bekannter Weise O,O-Diethyl-thionophosphorsäurediesterchlorid mit ω-Cyan-2,5-dimethylacetophenon in Gegenwart eines Säureakzeptors und eines für die Umsetzung inerten Verdünnungsmittels reagieren läßt und das Reaktionsprodukt nach an sich bekannten Methoden aufarbeitet.

**Claims**

1. Endoparasiticidal agent, characterised in that it contains at least one compound of the general formula I

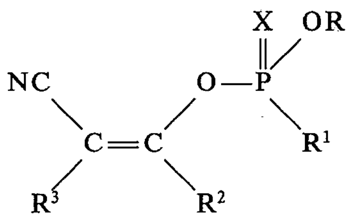

(I)

in which

X represents oxygen or sulphur,
R represents $(C_1-C_4)$-alkyl,
$R^1$ represents $(C_1-C_4)$-alkyl, phenyl, $(C_1-C_4)$-alkoxy, $(C_1-C_4)$-alkylthio or $(C_1-C_4)$-alkylamino,
$R^2$ represents phenyl or naphthyl, optionally substituted by halogen, cyano or nitro or by optionally halogen-substituted substituents from the series comprising $(C_1-C_4)$-alkyl, $(C_1-C_4)$-alkoxy or $(C_1-C_4)$-alkylthio and
$R^3$ represents hydrogen or $(C_1-C_4)$-alkyl.

2. Process for the preparation of an endoparasiticidal agent, characterised in that at least one compound of the general formula I in Claim 1 ist mixed with inert, nontoxic, pharmaceutically suitable excipients.

3. Compounds of the general formula I

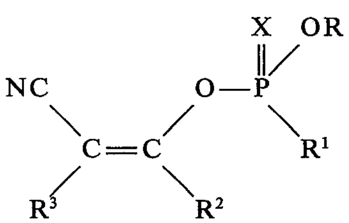

(I)

in which

X represents oxygen or sulphur,
R represents $(C_1-C_4)$-alkyl,
$R^1$ represents $(C_1-C_4)$-alkyl, phenyl, $(C_1-C_4)$-alkoxy, $(C_1-C_4)$-alkylthio or $(C_1-C_4)$-alkylamino,
$R^2$ represents phenyl or naphthyl, optionally substituted by halogen, cyano or nitro or by optionally halogen-substituted substituents from the series comprising $(C_1-C_4)$-alkyl, $(C_1-C_4)$-alkoxy or $(C_1-C_4)$-alkylthio and
$R^3$ represents hydrogen or $(C_1-C_4)$-alkyl,

for use in combating endoparasites.

4. Compound of the formula

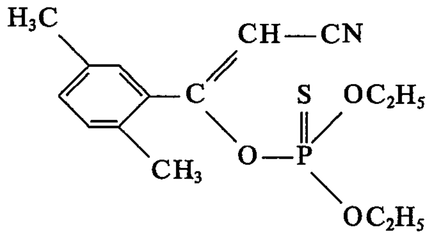

5. Process for the preparation of the compound of the formula according to Claim 4, characterised in that O,O-diethyl thionophosphoric acid diester chloride is allowed to react with ω-cyano-2,5-dimethylacetophenone in a manner which is in itself known, in the presence of an acid acceptor and a diluent which is inert for the reaction, and the reaction product is worked up by methods which are in themselves known.

**Revendications**

1. Composition endoparasiticide, caractérisée par une teneur en au moins un composé de formule générale I

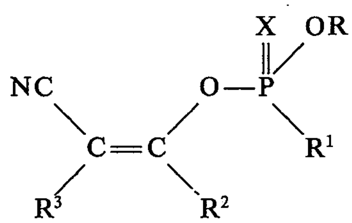

(I)

dans laquelle

- X représente l'oxygène ou le soufre,
- R est un groupe alkyle en $C_1$ à $C_4$,
- $R^1$ est un groupe alkyle en $C_1$ à $C_4$, phényle, alkoxy en $C_1$ à $C_4$, alkylthio en $C_1$ à $C_4$ ou alkylamino en $C_1$ à $C_4$,
- $R^2$ est un groupe phényle ou naphtyle éventuellement substitué par un halogène, un radical cyano ou nitro ou par des substituants, éventuellement substitués par un halogène, de la série alkyle en $C_1$ à $C_4$, alkoxy en $C_1$ à $C_4$ ou alkylthio en $C_1$ à $C_4$, et
- $R^3$ représente l'hydrogène ou un groupe alkyle en $C_1$ à $C_4$.

2. Procédé de production d'une composition endoparasiticide, caractérisé en ce qu'on mélange au moins un composé de formule générale I suivant la revendication 1 avec des supports inertes non toxiques, acceptables du point de vue pharmaceutique.

3. Composés de formule générale I

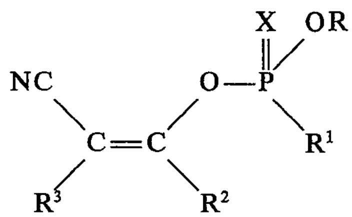

(I)

dans laquelle

- X représente l'oxygène ou le soufre,
- R est un groupe alkyle en $C_1$ à $C_4$,
- $R^1$ est un groupe alkyle en $C_1$ à $C_4$, phényle, alkoxy en $C_1$ à $C_4$, alkylthio en $C_1$ à $C_4$ ou alkylamino en $C_1$ à $C_4$,
- $R^2$ est un groupe phényle ou naphtyle éventuellement substitué par un halogène, un radical cyano ou nitro ou par des substituants, éventuellement substitués par un halogène, de la série alkyle en $C_1$ à $C_4$, alkoxy en $C_1$ à $C_4$ ou alkylthio en $C_1$ à $C_4$, et
- $R^3$ est l'hydrogène ou un groupe alkyle en $C_1$ à $C_4$,

destinés à être utilisés dans la lutte contre des endoparasites.

4. Composé de formule

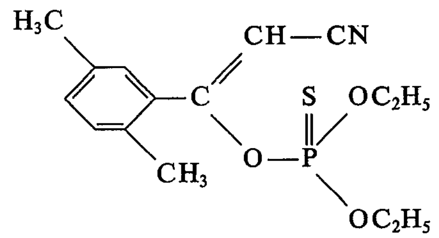

5. Procédé de préparation du composé de formule suivant la revendication 4, caractérisé en ce qu'on fait réagir d'une manière connue le chlorure du diester de O,O-diéthyle de l'acide thionophosphorique avec l'ω-cyano-2,5-diméthylacétophénone en présence d'un accepteur d'acide et d'un diluant inerte vis-à-vis de la réaction et on traite le produit réactionnel par des procédés connus.